(19) Europäisches Patentamt
European Patent Office
Office européen des brevets

(11) Publication number: **0 014 225**
**A1**

(12) # EUROPEAN PATENT APPLICATION

(21) Application number: 79100427.8

(22) Date of filing: 13.02.79

(51) Int. Cl.[3]: C 07 C 29/16
B 01 J 31/06, B 01 J 31/20
C 07 C 29/15

(43) Date of publication of application: 20.08.80 Bulletin 80/17

(84) Designated Contracting States: DE FR GB NL

(71) Applicant: THE DOW CHEMICAL COMPANY
2030 Abbott Road Post Office Box 1967
Midland, Michigan 48640(US)

(72) Inventor: Hartwell, George Edgar
585 Edmands Road
Framington Middlesex Massachusetts(US)

(72) Inventor: Garrou, Philip Ernest
93 Cottage Drive Ext.
Holliston Middlesex Massachusetts(US)

(74) Representative: Casalonga, Alain
Bureau D.A. Casalonga Lilienstrasse 77
D-8000 München 80(DE)

(54) **Amine-resin supported rhodium-cobalt bimetallic clusters as hydroformylation catalysts, their preparation and oxo process utilising such a catalyst.**

(57) Olefins are converted to alcohols by a one-step, hydroformylation process comprising contacting an olefin, such as 1-hexene, with a gaseous mixture of carbon monoxide and hydrogen in the presence of a catalyst prepared by loading a bimetallic cluster of the formula: $Rh_xCo_yCO_{12}$ wherein both x and y are each integers of 1-3 with the proviso that $\Sigma (x + y) = 4$, onto an amine resin.

EP 0 014 225 A1

TITLE MODIFIED
see front page

AMINE-RESIN SUPPORTED RHODIUM-COBALT
BIMETALLIC CLUSTERS AS NOVEL
HYDROFORMYLATION CATALYSTS

This invention relates to multinuclear metal catalysts. In one aspect, the invention relates to said catalysts supported by an amine resin. In other aspects, the invention relates to a method of preparing and to various methods of employing these supported catalysts.

The art-recognized method (I) of preparing alcohols from olefins is a two-step oxo, or hydroformylation, process:

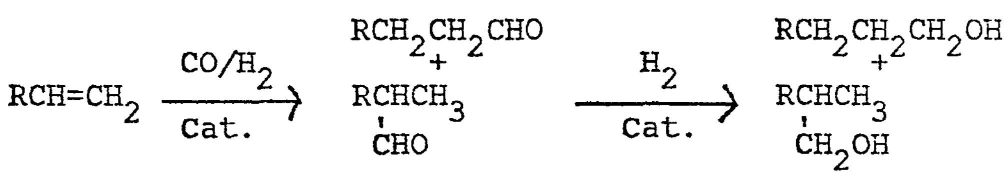


(I)

The first step comprises contacting at elevated temperature and pressure, e.g., 125°C-175°C and about 3000-5000 psi (212-353 kg/cm$^2$), an olefin with synthesis gas (a gaseous mixture of hydrogen and carbon monoxide) in the

presence of an oxo catalyst to produce a mixture of aldehydes. These aldehydes are then hydrogenated (typically in the presence of a hydrogenation catalyst) in a second step to their corresponding alcohols. Cobalt and rhodium are frequently used as the oxo catalysts.

Reported efforts to convert this two-step process to a highly selective one-step process have been singularly unsuccessful. Slaugh et al., J. Organometal. Chem., 13, 469 (1968), teach that a catalyst comprising cobalt carbonyl and a modifying ligand of tributyl phosphine affords a one-step, oxo synthesis of alcohols but accompanying olefin hydrogenation reduces yields (77 percent alcohol). Jurewicz et al., Adv. Chem. Ser., 132, 240-51 (1974) and Rollmann et al., Ger. 2,357,645, teach, respectively, that rhodium carbonyl supported on an amine resin or in the presence of tertiary amines also catalyzes a one-step oxo synthesis of alcohols. However, both research teams report suppressed yields (about 28 percent alcohol).

The defects of the prior art have been substantially overcome by the present invention, which is a catalyst comprising a rhodium-cobalt carbonyl bimetallic cluster supported on an amine resin.

The present invention is also directed to a method for preparing the rhodium-cobalt carbonyl catalyst.

The present invention also includes a one-step oxo process for converting olefins to alcohols, the process comprising reacting the olefin with a gaseous mixture of carbon monoxide and hydrogen at a temperature of at least 50°C and a pressure of at least 500 psi (35.3 $kg/cm^2$)

in the presence of a catalytic amount of a catalyst consisting of a rhodium-cobalt bimetallic cluster supported on an amine resin.

These catalysts are both easily prepared (by reacting the bimetallic cluster with an amine resin in an inert liquid medium) and exhibit long life. Also, these catalysts are highly selective in that alcohols are produced in essentially quantitative yield and permit the oxo process to be conducted at less rigorous process conditions.

The supported catalysts here used are prepared by loading onto an amine resin a bimetallic cluster of the formula:

$$Rh_xCo_yCO_{12} \qquad (II)$$

wherein x and y are each integers of 1-3 and x + y = 4. These bimetallic clusters are tetranuclear carbonyls and are readily synthesized by any number of varying methods. For example, tetranuclear carbonyl $Rh_2Co_2(CO)_{12}$ can be prepared by either of the following methods (III, IV):

$$Zn[Co(CO)_4]_2 + Rh_2(CO)_4Cl_2 \xrightarrow{\text{toluene}} Rh_2Co_2(CO)_{12} + ZnCl_2$$

(III)

$$2[Rh(CO)_2Cl_2]^- + 2[Co(CO)_4]^- \xrightarrow{H_2O} Rh_2Co_2(CO)_{12} + 4Cl^-$$

(IV)

These and other methods for preparing the bimetallic clusters of this invention are further described by Martinengo et al., J. Organometal. Chem., 59, 379 (1973). Conventional methods for preparing the monometallic carbonyls, e.g., $Zn[Co(CO)_4]_2$, etc., are described by King, Organometallic Synthesis, 1, 98-101 (1965). Clusters wherein x and y are each 2 are preferred.

Any amine resin that can be loaded with the bimetallic cluster (II) can be used in the practice of this invention. These resins are typically cross-linked and essentially water-insoluble, and they can possess primary, secondary and/or tertiary integral and/or pendant amine functionality. By "integral" amine functionality is meant that the amine functionality, i.e., amino group, is incorporated directly into the resin matrix. Examples of such resins include: Polyethylenepolyamine cross-linked with epichlorohydrin, urea-formaldehyde cross-linked copolymers and melamine-formaldehyde cross-linked copolymers. By "pendant" amine functionality is meant that the amine functionality is suspended from the resin matrix (backbone). This resin backbone can be essentially any cross-linked composition, such as styrene-divinylbenzene, styrene-glycoldimethacrylate, aniline-formaldehyde, aryl/polyamine-formaldehyde, phenol-formaldehyde, or polyacrylate. The pendant amine functionality can include such diverse functionality as primary, secondary and tertiary amines, di-, tri- and polyamines and hydrolyzed oxazolines. The preferred resins are weak-base anion exchange resins.

The amine resins of this invention can take many forms, but swellable gel or macroporous beads are the most common and are thus preferred. Resins having

pendant amine functionality are preferred to resins having integral amine functionality and resins having an exchange capacity of at least about 3 milliequivalents per gram of dry resin are particularly preferred. These latter resins include resins comprising a cross-linked polymer matrix having pendant amine functionality of the formulae $-N(R)_2$ and/or $-NHR'N(R)_2$ wherein each R is hydrogen or $C_1$-$C_6$ alkyl, R' is $C_2$-$C_6$ alkylene, and the open valence is the bond that joins the functionality to the polymer matrix. Typical substituents include: R alkyls, such as methyl, ethyl, propyl, isopropyl, butyl, etc.; and R' alkylenes, such as ethylene, propylene, hexylene, etc. The radical $-NHR'N(R)_2$ represents various diamines and alkyl-substituted diamines such as:

| $-NHR'N(R)_2$ | R' | $N(R)_2$ |
|---|---|---|
| ethylenediamine | $-CH_2CH_2-$ | $NH_2$ |
| propylenediamine | $-CH_2CH_2CH_2-$ | $NH_2$ |
| hexylenediamine | $-CH_2(CH_2)_4CH_2-$ | $NH_2$ |
| N-methylethylene-diamine | $-CH_2CH_2-$ | $NHCH_3$ |
| N,N-dimethyl-ethylenediamine | $-CH_2CH_2-$ | $N(CH_3)_2$ |
| N,N-ethylmethyl-ethylenediamine | $-CH_2CH_2-$ | $N(CH_3)(CH_2CH_3)$ |

The amine functionality of these preferred resins can be either primary, secondary and/or tertiary although primary functionality is preferred when the amine is of the formula $-NHR'N(R)_2$ and secondary and tertiary functionality is preferred when the amine is of the formula $-N(R)_2$. $-N(R)_2$ functionality is preferred to $-NHR'N(R)_2$ functionality. An especially preferred, commercial amine

resin is a macroporous, cross-linked polystyrene polymer matrix having pendant amine functionality of the formula $-N(R)_2$ wherein each R is methyl.

The catalyst of this invention is prepared by loading the bimetallic cluster onto the amine resin. This loading is accomplished by a method comprising reacting in an inert liquid medium the bimetallic cluster with the amine resin. Typically, the reaction is conducted at a cluster:amine on resin mole ratio (i.e., cluster:resin mole equivalents ratio) of at least about 1:100 and preferably at least about 1:25. The maximum cluster:resin mole ratio equivalents ratio can be varied as desired but is typically about 1:1 and preferably about 1:4.

By "inert liquid" is meant one that is non--reactive with the materials used, under the conditions employed.

The inert liquid comprises one or more liquid solvent(s) in which the bimetallic cluster is soluble and which is inert (non-reactive) with both the reagents and products. Aliphatic and aromatic hydrocarbons and substituted hydrocarbons are illustrative solvents and include such compounds as hexane, benzene, toluene, o-, m- and p-xylenes, o-dichlorobenzene, methylene chloride, chloroform and carbon tetrachloride. Benzene and the alkyl--substituted aromatic hydrocarbons having up to 3 substituent alkyl groups having not more than 2 carbon atoms in each alkyl group are preferred, with benzene and toluene especially preferred.

This method can be practiced at any temperature and pressure at which the reaction mixture of bimetallic cluster and solubilizing inert liquid are liquid. It is most convenient to use ambient temperature and pressure, e.g., 20°C-30°C and atmospheric pressure. The reaction is typically conducted under an inert atmosphere, such as argon, and for a sufficient period of time to load the bimetallic cluster onto the resin. This period of time will vary with the reagents and conditions employed, but with many reagents and at ambient conditions the loading is partially achieved after about 2 hours, and is generally complete after about 12 hours. The resulting catalyst is recovered by any convenient physical separation technique, e.g., filtering.

The physical and chemical structure of the catalyst, i.e., the bimetallic cluster loaded (supported) upon the amine resin, is not fully known. However, it is known that the catalyst contains rhodium and cobalt carbonyl attached to the amine resin. Typically, the catalyst comprises, as determined by any conventional elemental analysis method, at least about 1 weight percent, and preferably at least about 4 weight percent, rhodium (metal basis) and at least about 0.5 weight percent, and preferably at least about 2.5 weight percent, cobalt (metal basis). Resin saturation is the only limitation upon the maximum weight percent of rhodium and cobalt and the saturation levels are preferred for reasons of catalytic activity and life. Saturation levels vary dependent upon the particular amine resin and bimetallic cluster employed.

These new catalysts are used in substantially the same manner as known catalysts. This invention's oxo process of converting olefins directly to alcohols requires a catalytic amount of catalyst. Typically, the minimum amount of catalyst (Rh-Co metal basis) present is about 0.1 weight percent, and preferably about 0.25 weight percent, based on the weight of olefin. Practical considerations such as economy and convenience are the only limitations upon the maximum amount of catalyst that can be present, although preferably a maximum amount of about 5 weight percent, and most preferably of about 2 weight percent is used. Conventional oxo process (reaction) times are employed.

While these catalysts are operable under typical oxo temperatures and pressures, i.e., about 125°C-175°C and 3000-5000 psi (212-353 $kg/cm^2$), they are also operable at much lower temperatures and/or pressures. Accordingly, in the presence of the catalysts here used, olefins are directly converted to alcohols at conditions of at least about 50°C and at least about 500 psi (35.3 $kg/cm^2$). However, in most instances best results are achieved at conditions of at least about 100°C and 750 psi (53 $kg/cm^2$) and thus these conditions are preferred.

Any olefin that can be converted to an aldehyde by the known oxo process can be converted to an alcohol by this invention's oxo process. Illustrative oxo olefins include: Ethylene, propylene, 1-butene, 2-butene, 1-pentene, 1-hexene, 1-decene, 3-methyl-1-butene, butadiene, 1,4-pentadiene, isoprene, cyclohexene, cycloheptene, dicyclopentadiene, norborene, allyl alcohol and allyl acetate. Suitable combinations of different olefins, such as ethylene and propylene, can also be used.

Any conventional gaseous mixture of hydrogen and carbon monoxide can be here used although amounts in excess of stoichiometric hydroformylation process requirements are preferred. Typically, the hydrogen:carbon monoxide ($H_2$:CO) mole ratio here used varies from 1:10 to 10:1, preferably about 1:1.

The catalysts and hydroformylation process described herein are especially adaptable to preparing dimethanoldicyclopentadiene from dicyclopentadiene (DCPD). The term "dicyclopentadiene (DCPD)" as used herein includes both crude and purified DCPD. Crude DCPD is a hydrocarbon stream, such as that taken from a naphtha or LPG cracker, containing at least about 50 percent and normally not more than about 95 weight percent DCPD. It is to be understood that the catalysts and hydroformylation process described herein are applicable to DCPD, whether it be the normal crude or the purified material.

The following examples illustrate this invention. Unless indicated otherwise, all parts and percentages are by weight.

<u>Example 1(a) Catalyst Preparation</u>

Beads (0.25 g) of an amine resin having a cross--linked polystyrene backbone and pendant benzyl dimethylamine functionality, a bimetallic cluster of the formula $Rh_2Co_2(CO)_{12}$ (0.2 g) and toluene (20 ml) were mixed and agitated under argon at ambient conditions for 12 hours. The thus-modified beads (catalyst) were then filtered and dried under vacuum. The catalyst had a gray-black color when wet with toluene and a grayish color when dry. Elemental analysis showed the catalyst to contain 4 weight

percent rhodium and 2.6 weight percent cobalt, and an infrared spectrum exhibited several overlapping strong absorptions at about 1890 $cm^{-1}$, a strong absorption at about 2000 $cm^{-1}$ and a medium absorption at about 2010 $cm^{-1}$ in the carbonyl stretching frequency region.

Example 1(b)

The catalyst (0.2 g) thus prepared was charged to a pressure reactor with dry, deaerated benzene (3 ml) under argon. 1-Hexene (1 ml) was then added and the reactor contents subsequently flushed with nitrogen and pressured with equimolar amounts of carbon monoxide and hydrogen. The reactor was heated to 100°C, with a pressure of 750 psi (53 $kg/cm^2$), and held thereat for 2 hours. The reactor was then allowed to cool and the contents analyzed by both gas chromatography (GC) and nuclear magnetic resonance (NMR). The reaction product contained 96.3 percent alcohol.

Control A

A catalyst was prepared according to the above procedure except that $Rh_4(CO)_{12}$ was substituted for the bimetallic cluster and the catalyst comprised 9.2 percent Rh rather than 6.6 percent Rh-Co. Example 1(b) was then repeated except that the catalyst thus prepared was substituted for the catalyst prepared in Example 1(a) and a pressure of 1,000 psi (71 $kg/cm^2$) was substituted for 750 psi (53 $kg/cm^2$). GC analysis of the reaction product showed aldehydes present to the extent of 21 percent and alcohols present to the extent of only 54.4 percent.

Control B

A catalyst was prepared according to the procedure of Example 1(a) except that $Co_4(CO)_{12}$ was substituted for the bimetallic cluster and the catalyst comprised 3 percent Co rather than 6.6 percent Rh-Co. Example 1(b) was then repeated except that the catalyst thus prepared was substituted for the catalyst prepared in Example 1(a) and a pressure of 1,000 psi (71 kg/$cm^2$) was substituted for 750 psi (53 kg/$cm^2$). GC analysis of the reaction product showed aldehydes present to the extent of 71.1 percent with only 1.2 percent alcohol present.

The results of Controls A and B demonstrate the marked difference between supported Rh and Co monometallic clusters and supported Rh-Co bimetallic clusters.

Control C

A catalyst was prepared according to the procedure of Example 1(a) except that $Rh_6(CO)_{16}$ and $Co_2(CO)_8$ at a 1:1 metal ratio were substituted for the bimetallic cluster. Example 1(b) was then repeated except that the catalyst thus prepared was substituted for the catalyst prepared in Example 1(a), a pressure of 1,000 psi (71 kg/$cm^2$) was substituted for 750 psi (53 kg/$cm^2$), and a residence time of 3 hours was substituted for 2 hours. GC analysis of the reaction product showed aldehydes present to the extent of 83 percent. This demonstrates the great contrast between a catalyst prepared from an Rh-Co bimetallic cluster and a catalyst prepared from individual Rh and Co carbonyls.

Control D

Following a procedure similar to Example 1(b), 1-hexene (1 ml) was charged to a reactor containing dry deaerated benzene (3 ml) and a mixture of benzyl dimethylamine and $Rh_2Co_2(CO)_{12}$. The mixture comprised about 6.5 weight percent metal as did the catalyst of Example 1(b). The reactor contents were then flushed with nitrogen and pressured with equimolar amounts of carbon monoxide and hydrogen and heated to 100°C, the pressure reaching 1,000 psi (71 kg/cm$^2$). The reactor contents were held thereat for 3 hours, allowed to cool and subsequently analyzed. The reaction product contained 71.9 percent aldehydes, 12.1 percent alcohols and the remainder was a mixture of isomerization products. This demonstrates the marked difference between an Rh-Co bimetallic cluster supported by an amine resin and an Rh-Co bimetallic cluster merely in the presence of amine functionality.

Examples 2-4

Control D was repeated except the catalyst of Example 1(a) was substituted for the catalyst of Control D and various olefins were substituted for 1-hexene. The results are tabulated below.

TABLE I

Olefins to Alcohols via Oxo Process Catalyzed by $Rh_2Co_2(CO)_{12}$

| Ex | Olefin | Conversion (%) | Alcohol (%) | Aldehyde (%) |
|---|---|---|---|---|
| 2 | Cyclohexene | 78 | 85 | 0 |
| 3 | Allyl Alcohol | 100 | 100 | 0 |
| 4 | Allyl Acetate | 100 | 100 | 0 |

The product alcohols of Examples 3 and 4 included cleavage products, i.e., some allyl alcohol and allyl acetate underwent hydrogenolysis (V) to propene and propene was then hydroformylated (VI).

$$H_2C{=}CHCH_2CH_2OH \xrightarrow{H_2} H_2C{=}CHCH_3 + H_2O \qquad (V)$$

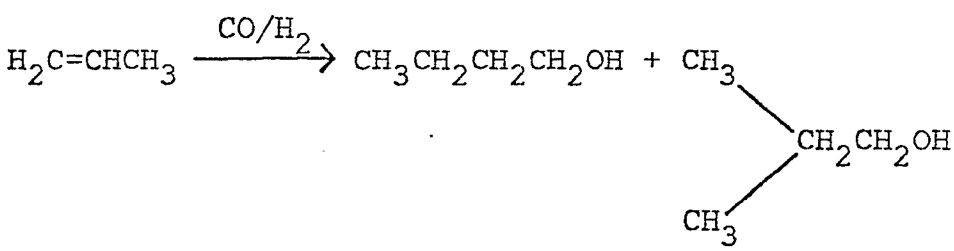


(VI)

Example 5

The procedure of Example 1(b) was again repeated except toluene was substituted for benzene, dicyclopentadiene was substituted for 1-hexene and the reactor contents were heated to 125°C with the pressure reaching 1,500 psi (106 kg/cm$^2$) and the contents were held thereat for 5 hours. Analysis showed a quantitative conversion of the olefin to dimethanoldicyclopentadiene.

# CLAIMS

1. A catalyst comprising a rhodium-cobalt carbonyl bimetallic cluster supported on an amine resin.

2. The catalyst of Claim 1 prepared by loading onto an amine resin a bimetallic cluster of the formula:

$$Rh_xCo_y(CO)_{12}$$

wherein x and y are each integers of 1-3 and x + y = 4.

3. The catalyst of Claim 2 comprising at least 1 weight percent rhodium and at least 0.5 weight percent cobalt.

4. The catalyst of Claim 3 wherein the amine resin comprises cross-linked, essentially water-insoluble swellable gel or macroporous beads.

5. The catalyst of Claim 4 wherein the amine resin has an exchange capacity of at least 3 milliequivalents per gram of dry resin.

6. The catalyst of Claim 5 wherein the amine resin comprises a cross-linked polymer matrix having pendant amine functionality of the formulae $-N(R)_2$ and/or $-NHR'N(R)_2$ wherein each R is hydrogen or $C_1$-$C_6$ alkyl, R' is $C_2$-$C_6$ alkylene, and the open valence is the bond that joins the functionality to the polymer matrix.

7. The catalyst of Claim 1 wherein the amine resin comprises a cross-linked polystyrene polymer matrix and each R is methyl.

8. A method of preparing the catalyst of Claim 1, the method comprising reacting in an inert, liquid medium a rhodium-cobalt carbonyl bimetallic cluster with an amine resin.

9. The method of Claim 8 wherein the bimetallic cluster and amine resin are contacted at a cluster:resin mole equivalents ratio of at least 1:100.

10. A one-step oxo process for converting olefins to alcohols, the process comprising reacting the olefin with a gaseous mixture of carbon monoxide and hydrogen at a temperature of at least 50°C and a pressure of at least 500 psi (35.3 $kg/cm^2$) in the presence of a catalytic amount of a catalyst consisting of a rhodium-cobalt bimetallic cluster supported on an amine resin.

11. The process of Claim 10 wherein the contacting is conducted at a temperature of at least 100°C and a pressure of at least 750 psi (53 $kg/cm^2$).

12. The process of Claim 10 wherein the olefin is dicyclopentadiene.

0014225

Application number

European Patent Office

# EUROPEAN SEARCH REPORT

EP 79 100 427.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.3) |
|---|---|---|---|
| A | DE - A1 - 2 727 245 (BRINTZINGER) * claims 1 and 11 * -- | 1 | C 07 C 29/16<br>B 01 J 31/06<br>B 01 J 31/20<br>C 07 C 29/15 |
| A | US - A - 3 929 969 (BROWN) * columns 9 to 10, table, example 1 * -- | 2 | |
| A | US - A - 3 890 28? (ANGSTADT et al.) * claims 1, 6 and 7 * -- | 1,6 | |
| D | Chemical Abstracts, Volume 82, Nr. 9, March 3, 1975, (COLUMBUS, OHIO, USA) A.T. JUREWICZ et al. "Hydroformylation with rhodium-amine complexes" page 528, column 1, abstract Nr. 57 172 E & Adv. Chem. Ser. Volume 132, 1974, pages 240 to 251 (Eng) ---- | 1,6 | |

TECHNICAL FIELDS SEARCHED (Int. Cl.3)

B 01 J 31/00
C 01 G 1/04
C 07 C 29/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 04-10-1979 | KNAACK |

EPO Form 1503.1 06.78